# EUROPEAN PATENT APPLICATION

(11) **EP 2 008 668 A1**
(43) Date of publication of application: **31.12.2008**
(21) Application number: 08011544.7
(22) Date of filing: 25.06.2008
(51) Int. Cl.: A61L 2/08, C12P 7/10, C10G 1/00

(54) **Method for producing biofuel using electron beam**

(30) Priority: 25.06.2007 KR 20070062314
(71) Applicant: Korea Accelerator & Plasma Research Association, Cheorwon-gun Gangwon-do 269-843 (KR)
(72) Inventor: Lee, Kang Ok, Seoul 139-200 (KR); Chung, Kie Hyung, Gyeonggi-do 450-050 (KR)
(74) Representative: Hano, Christian

(57) **Abstract**

A method for producing biofuel using an electron beam is disclosed. The method for producing biofuel includes: irradiating an electron beam to the vegetable polymer to decompose and sterilize the vegetable polymer, thereby promoting conversion of the vegetable polymer to biomass compared to the traditional methods.

## Description

### Technical Field

The present invention relates to a method for producing biofuel, and more particularly, to a method for producing biofuel using an electron beam.

### Background Art

Biofuel is a sustainable energy source produced from a biomass which exists in the natural world. A biomass is a general term of organic matters of living organisms, and its kind is very various like, for example, a byproduct generated in various kinds of plants and animals and in agriculture and forestry, waste, and food waste, industrial waste based on a living organism, and crops (energy crops) cultivated to produce biofuel.

A biomass can be converted into biofuel of a solid state, a liquid state or a gaseous state by using physical, chemical and biological techniques. As a representative example of biofuel, there are bio-ethanol, bio-buthanol, bio-diesel, bio-gas, and other solid fuels. The biofuels can be used for generation of an electric power and transportation, but biofuel for transportation is actively being developed due to an advantage of mobility and an environmental characteristic.

Bio-ethanol is usually produced by fermenting a starch of maize or sugar cane, and bio-diesel is usually extracted from beans or rape seed oil. Due to advantages that biofuel is not greatly lower in efficiency than fossil fuel, have an environment-friendly characteristic and can also use the existing fuel infrastructures "as is", it attracts public attention as an alternative energy source.

Biofuel can greatly reduce emission of contaminants such as fine particles and sulfur compounds compared to fossil fuel. Carbon oxide emitted from biofuel is absorbed by and fixed to plants, and so pure emission of carbon oxide does not almost occur. Emission of sulfur compounds or hydrocarbon is significantly reduced compared to fossil fuel, and so an occurrence of smog and ozone can be greatly reduced, so biofuel is very suitable for vehicle fuel.

An alternative energy technique for producing alcohol by fermenting an alcohol spawn of grain has been developed as a national industry in Brazil, USA, Europe, Denmark, and Indonesia, and a development of an alternative energy for preparing for a reduction of fossil fuel after half a century has been risen as a national problem.

First generation biofuel has been researched by using an animal feed or by focusing corn-based ethanol based on grain such as corn. However, the amount of produced grain is restrictive to satisfying an increasing demand on ethanol, and so there is a problem in that it is difficult to continuously produce. In case of USA, about 15% of produced corn is used to produce ethanol, and so there is a possibility of a food shortage. Also, even though the whole amount of corn produced in USA is used, merely 20% of gasoline is replaceable with ethanol.

Second generation biofuel has been developed focusing on cellulose-based ethanol based on cellulose extracted from wood which does not consume a food resource. USA can produce ethanol of about 50 billion gallons per year from various kinds of agriculture byproducts, and a possibility of a technique improvement is high and it is more profitable in manufacturing cost compared to corn-based ethanol, so it is attracting public attention as next generation biofuel.

However, wood-based biomass has a strong covalent bond with high bond energy. That is, since cellulose is not easy to hydrolyze and lignin and hemicellulose are bad in accessibility, a manufacturing process is lengthy and so there is an urgent need for a manufacturing process that mass production is possible.

### Disclosure of Invention

### Technical Problem

The present invention is directed to a method for producing biofuel in which cellulose-based biofuel using wood which is a sustainable energy source is efficiently produced.

### Technical Solution

The present invention provides a method for producing biofuel using an electron beam, comprising: irradiating an electron beam to the vegetable polymer to decompose and sterilize the vegetable polymer, thereby promoting conversion of the vegetable polymer to biomass.

The vegetable polymer comprises cellulose and lignin. The electron beam comprises a gamma ray, an x ray, and an ion-beam.

According to the present invention, the processing time for converting the vegetable polymer into biofuel is significantly reduced by processing the vegetable polymer using the electron beam to change a geometrical structure of cellulose and lignin which composes the vegetable polymer. Accordingly, mass production of bio-energy which can replace fossil fuel is possible.

### Brief Description of Drawings

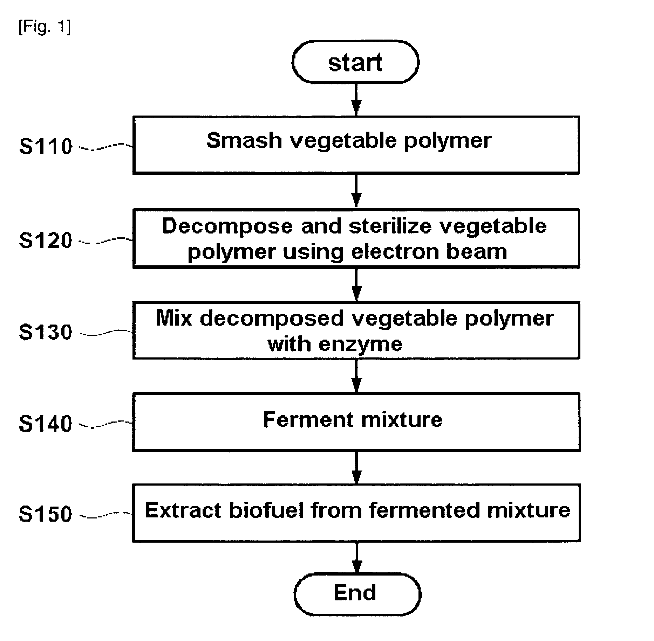

FIG. 1 is a flowchart illustrating a method for producing biofuel using an electron beam according to an exemplary embodiment of the present invention.

### Best Mode for Carrying Out the Invention

Hereinafter, exemplary embodiments of the present invention will be described in detail. However, the present invention is not limited to the exemplary embodiments disclosed below, but can be implemented in various types. Therefore, the present exemplary embodiments are provided for complete disclosure of the present invention and to fully inform the scope of the present invention to those ordinarily skilled in the art.

FIG. 1 is a flowchart illustrating a method for producing biofuel using an electron beam according to an exemplary embodiment of the present invention.

Referring to FIG. 1, a pre-processing step for decomposing a vegetable polymer by using an electron beam is performed. For example, the vegetable polymer is smashed (S110). The vegetable polymer comprises wood containing cellulose and lignin, a herbaceous plant, a product or waste wood derived from a herbaceous plant, or a paper. As a smashed form of the vegetable polymer, there are a wood chip which is pieces of wood cut by a crusher or a wood pellet of a cylindrical shape made by compressing sawdust generated during wood processing.

A process for converting the vegetable polymer into a biomass is necessary in order to extract biofuel from the vegetable polymer such as wood or grass. Most of vegetable polymers have a strong covalent bond, and thus it is not easy to convert it into a biomass.

In more detail, a chemical composition of wood depends on a kind of tree, and so in order to systemize the chemical composition, an extract is classified as a minor component, and a cell wall substance is classified as a major component. A component which composes the cell wall substance comprises cellulose, hemicellulose and lignin. Cellulose is a homopolymer of β-[1,4] D-glucose molecules linked in a linear chain and has a chemical formula of (C₆H₁₀O₅)n. Cellulose is strong to alkali, but it is hydrolyzed into glucose by an acid.

Hemicellulose is obtained by subtracting a pectic substance from polysaccharide and composes a root, a rhizome, a seed, and a cell of a fruit. It is extracted from a cell wall in which a pectic substance is removed by using an alkali solution, and its major components are xylan (formed such that many molecules of xylose which is pentose are bound), glucan, xyloglucan, and glucomannan. Lignin is a component which composes a wood substance together with cellulose and hemicellulose. A chemical structure of lignin is not clearly known but inferred to be between C₁₈H₂₄O₁₁ and C₄₀H₄₅O₁₈.

An electron beam is irradiated to the smashed vegetable polymer having such components, so that the vegetable polymer is decomposed and sterilized (S120). The electron beam comprises a gamma-ray, an x-ray, and an ion-beam. When the electron beam is irradiated to the vegetable polymer, part of the vegetable polymer can be converted into sucrose or starch in a short time.

In more detail, all materials are composed by a bond between atoms or molecules, and if an accelerated electron is irradiated to the bond, the bond between molecules is easily broken. If an electron beam of high energy is irradiated to a material by using this principle, a chemical structure of a material changes by the energy, thereby raising a value of a material or extincting or removing harmful bacteria. That is, a polymer component of the vegetable polymer can be decomposed into monomers by using the electron beam.

A bond between molecules of cellulose, hemicellulose and lignin is broken by the electron beam, so that the vegetable polymer can be rapidly decomposed. For example, the electron beam having energy of 2MeV to 10MeV is irradiated to the smashed vegetable polymer with a dose of tens to hundreds of kGy during several to tens of minutes. As the energy intensity of the electron beam is high, an effect of decomposition and sterilization is more increased, but in case of an industrial electron beam accelerator, the intensity is limited to equal to or less than 10MeV by a European standard (EN552), and thus the intensity of the electron beam is preferably equal to or less than 10MeV.

The electron beam has a characteristic that the processing thickness depends on density (0.35 cm/MeV based on density of "1 "), and an x-ray is generated when it interacts with a material, and it is similar to a characteristic (24cm based on density of "1") of a gamma ray with a strong material penetrating force. The electron beam has a relatively large irradiation area compared to a gamma ray and can significantly reduce a processing time, and it can also vary the energy. In case of a gamma ray, emission energy of usually used nuclide Co₆₀ is 1.17MeV to 1.33MeV (average 1.25MeV), and emission energy of nuclide Cs₁₃₇ is 0.667MeV which is constant.

As described above, by using the electron beam, in addition to a characteristic of an electron beam, an x-ray or a gamma ray which is additionally generated contributes to a reaction to compensate the disadvantage of the processing thickness. Therefore, the processing time can be significantly reduced.

In addition, bacteria which exist on a raw material surface composed of the vegetable polymer can be sterilized. Productivity of biofuel such as alcohol can be improved by having an enzyme and strain put in biofuel production to work smoothly. It is because in case of grain, about 25% is contaminated by mycotoxin which disturbs a yeast growth, so that a yeast growth is suppressed to obstruct biofuel production.

The electron beam can be generated by the electron beam accelerator. The electron beam accelerator condenses thermal electrons emitted from a cathode by a strong magnetic field to convert electrons of low energy into an electron beam of high energy through an accelerating tube. The condensed electron beam passes through a scanning horn and is uniformly irradiated to a target material. A radiation dose for the target material is controlled by adjusting a radiation time using a conveyor. The electron beam accelerator is turned on or off by a comparison with a gamma ray continuously emitted from nuclide. Thus, since an electron beam can be generated only when needed, it is easy to maintain and operate, and it is possible to easily and precisely adjust a radiation dose.

As described above, compared to the pre-processing process using a conventional decomposition process such as a thermal decomposition, the processing time is significantly reduced, leading to a sterilization effect. That is, there is no need for an additional sterilizing process which is necessary in the conventional art.

Next, a step for fermenting the decomposed vegetable polymer to extract biofuel is performed. The decomposed vegetable polymer and the enzyme are mixed (S130). For example, the decomposed vegetable polymer, the enzyme containing saccharifing amylase such as an microzyme (S. cerevisiae IFO, S. formosensis nov, S. robustus nov.sp, and etc.), zymomonas mobilis, and rhizozyme, and strain are mixed.

Then, the mixture of the vegetable polymer and the enzyme is fermented (S140). In order to promote ferment of the mixture, an appropriate temperature, an appropriate pressure and an appropriate electromagnetic wave are preferably provided. For example, the mixture of the vegetable polymer and the enzyme is fermented at a temperature of 15°to 45°, under appropriate humidity and pressure, and in atmosphere of oxygen (O₂), hydrogen (H₂) and amino nitrogen. Here, in order to promote ferment of the mixture, a spawn and sterile water can be additionally inserted. As described above, since saprophyte is sterilized by irradiation of the electron beam, the fermenting process can be efficiently performed.

Finally, biofuel is extracted from the fermented mixture (S150). A technique for extracting biofuel from the fermented mixture is well known to a person having ordinary skill in the art. As extractable biofuel, there are gas, bio-ethanol, and bio-butanol.

As described above, the method for producing biofuel using the electron beam according to the present invention irradiates the electron beam to the vegetable polymer to thereby sterilize bacteria and promote saccharification of cellulose and lignin which are not easy to decompose. Also, if the enzyme is added and a condition of an appropriate temperature, humidity and pressure of gas such as oxygen is given, it is converted into biomass like sucrose from which biofuel is extracted.

As a result, the processing time of the biofuel producing process is significantly reduced, and second generation alternative energy which reduces the global warming effect can be efficiently produced, and thus use of fossil fuel is reduced, leading to a tremendous economic pervasive effect.

## Claims

1. A method for producing biofuel using an electron beam, comprising: irradiating an electron beam to the vegetable polymer to decompose and sterilize the vegetable polymer, thereby promoting conversion of the vegetable polymer to biomass.

2. The method of claim 1, wherein the vegetable polymer comprises cellulose and lignin.

3. The method of claim 1, wherein the electron beam comprises a gamma ray, an x ray, and an ion-beam.
